# EUROPÄISCHE PATENTSCHRIFT

(11) **EP 3 597 571 B1**
(45) Veröffentlichungstag und Bekanntmachung des Hinweises auf die Patenterteilung: **17.08.2022**
(21) Anmeldenummer: 19181953.1
(22) Anmeldetag: 24.06.2019
(51) Int. Cl.: B65G 47/76, B65G 43/08, B30B 15/32, B30B 11/00, B30B 11/08

(54) **TABLETTENABLAUF EINER TABLETTENPRESSE SOWIE VERFAHREN ZUM BETÄTIGEN EINER WEICHE EINES TABLETTENABLAUFS**
TABLET OUTLET OF A TABLET PRESS AND METHOD FOR ACTUATING A SWITCH OF A TABLET OUTLET
SORTIE DE COMPRIMÉS D'UNE PRESSE À COMPRIMÉS AINSI QUE PROCÉDÉ DE COMMANDE D'UN AIGUILLAGE D'UNE SORTIE DE COMPRIMÉS

(30) Priorität: 04.07.2018 DE 102018116202
(43) Veröffentlichungstag der Anmeldung: 22.01.2020
(73) Patentinhaber: Fette Compacting GmbH, 21493 Schwarzenbek (DE)
(72) Erfinder: Meißner, Friedrich, 21493 Schwarzenbek (DE); Lüdemann, Stefan, 21035 Hamburg (DE)
(74) Vertreter: Hauck Patentanwaltspartnerschaft mbB

(56) Entgegenhaltungen:
- EP-A2- 2 368 704
- WO-A1-2008/038070
- CN-U- 205 112 500

## Beschreibung

Die Erfindung betrifft einen Tablettenablauf einer Tablettenpresse, insbesondere einer Rundläufertablettenpresse, mit einer in dem Tablettenablauf angeordneten Weiche, wobei eine Steuereinrichtung vorgesehen ist, und wobei eine Antriebseinrichtung vorgesehen ist, die die Weiche abhängig von einem Schaltsignal der Steuereinrichtung zwischen einer ersten Schaltstellung, in der Tabletten einem ersten Tablettenausgang zugeleitet werden, und mindestens einer zweiten Schaltstellung, in der Tabletten mindestens einem zweiten Tablettenausgang zugeleitet werden, bewegt, wobei mindestens ein Sensor vorgesehen ist zum Detektieren des Erreichens der ersten Schaltstellung und der mindestens einen zweiten Schaltstellung durch die Weiche.

Die Erfindung betrifft weiterhin eine Rundläufertablettenpresse, umfassend einen mittels eines Drehantriebs drehbaren Rotor, wobei der Rotor eine obere Stempelführung für obere Stempel der Rundläufertablettenpresse und eine untere Stempelführung für untere Stempel der Rundläufertablettenpresse sowie eine zwischen den Stempelführungen angeordnete Matrizenschreibe aufweist, wobei die Stempel mit Kavitäten der Matrizenscheibe zusammenwirken, weiter umfassend eine Fülleinrichtung, durch die zu verpressendes Füllmaterial in die Kavitäten der Matrizenscheibe gefüllt wird, weiter umfassend mindestens eine obere Druckeinrichtung und mindestens eine untere Druckeinrichtung, die im Betrieb mit den oberen Stempeln und mit den unteren Stempeln zum Verpressen des Füllmaterials in den Kavitäten der Matrizenscheibe zu Tabletten zusammenwirken, weiter umfassend eine Auswurfeinrichtung, in der in den Kavitäten erzeugte Tabletten ausgeworfen werden.

Die Erfindung betrifft außerdem ein Verfahren zum Betätigen einer Weiche eines Tablettenablaufs einer Tablettenpresse, insbesondere einer Rundläufertablettenpresse, bei dem die Weiche zwischen einer ersten Schaltstellung, in der Tabletten einem ersten Tablettenausgang zugeleitet werden, und mindestens einer zweiten Schaltstellung, in der Tabletten mindestens einem zweiten Tablettenausgang zugeleitet werden, bewegt wird, wobei mittels mindestens eines Sensors das Erreichen der ersten Schaltstellung und der mindestens einen zweiten Schaltstellung durch die Weiche detektiert wird.

Rundläufertablettenpressen weisen eine Vielzahl von oberen und unteren Stempeln auf, die jeweils paarweise einer Kavität einer Matrizenscheibe eines Rotors der Rundläufertablettenpresse zugeordnet sind. Im Zuge der Drehung des Rotors werden die Kavitäten mit dem zu verpressenden Füllmaterial befüllt. In mindestens einer Druckeinrichtung werden die oberen und unteren Stempel in den Kavitäten zum Verpressen des Füllmaterials zu Tabletten gegeneinander gedrückt. Nach dem Verpressen werden die Tabletten in der Regel durch die Unterstempel aus den Kavitäten ausgestoßen und beispielsweise durch einen Abstreifer einem Tablettenablauf zugeführt. Die abgeführten Tabletten werden beispielsweise anhand von Messwerten einer Sensorik der Rundläufertablettenpresse unterschiedlichen Tablettenausgängen zugeleitet, beispielsweise Tablettenausgängen für Guttabletten, für Schlechttabletten oder für Tabletten eines Musterzugs. Zum Leiten der Tabletten in unterschiedliche Tablettenausgänge befinden sich in Tablettenabläufen von Rundläufertablettenpressen regelmäßig Weichen. Solche Weichen können zum Beispiel eine schwenkbar zwischen beispielsweise zwei Schaltstellungen bewegbare Weichenfahne aufweisen, die die Tabletten je nach Schaltstellung zu einem ersten Tablettenausgang oder einem zweiten Tablettenausgang leitet.

Es ist bekannt, das Erreichen der jeweiligen Endlagen der Weiche mittels Sensoren zu detektieren. Für eine Weiche mit zwei Schaltstellungen sind in der Regel zwei Sensoren für das Detektieren der jeweiligen Endlage vorgesehen. Sofern ein Nichterreichen einer Endlage durch die Sensoren erkannt wird, wird eine entsprechende Fehlermeldung ausgegeben.

Entsprechend der hohen Produktionskapazitäten moderner Rundläufertablettenpressen fließt durch den Tablettenablauf ein erheblicher Strom von Tabletten. Dabei fließen die Tabletten regelmäßig mit nur geringem Abstand zwischen einander. Dies kann beim Schalten der Weiche in der Praxis zu einem Einklemmen von Tabletten zwischen der Weiche, beispielsweise dem freien Ende einer Weichenfahne, und einer gegenüberliegenden Wand des Tablettenablaufs führen. Die Weiche kann entsprechend ihre Zielschaltstellung nicht erreichen. Dies wiederum kann zu einem Tablettenstau in dem Tablettenablauf und zu einem Abschalten der Rundläufertablettenpresse aufgrund einer Weichenfehlfunktion führen. Dies schränkt die Verfügbarkeit der Rundläufertablettenpresse erheblich ein und der manuelle Aufwand für eine Bedienperson zum Beheben der Fehlfunktion ist hoch. So ist es für das Beheben eines Tablettenstaus erforderlich, das Gehäuse der Rundläufertablettenpresse zu öffnen, was insbesondere bei toxischen Pressmaterialien zu einem erhöhten Gesundheitsrisiko führt. Weiter ist regelmäßig eine Demontage von Komponenten, wie der Weichenantriebe, der Säule Luft, Abdeckungen etc. erforderlich, um den Bereich des Tablettenstaus zugänglich zu machen. Die gestauten Tabletten müssen entfernt werden, ebenso etwaige Bruchstücke und der Tablettenablauf muss gereinigt werden. Weiterhin muss geprüft werden, ob versehentlich Schlechttabletten oder Bruchstücke in die Gutproduktion gelangt sind. Anschließend müssen die demontierten Komponenten wieder montiert werden und die Rundläufertablettenpresse muss wieder in Betrieb genommen werden.

Aus WO 2008/038070 A1 ist ein Tablettenablauf mit einer darin angeordneten Weiche bekannt mit einer Steuereinrichtung und einer Antriebseinrichtung zum Bewegen der Weiche zwischen einer Ausgangsschaltstellung und einer Zielschaltstellung. WO 2008/038070 A1 offenbart ein Tablettenablauf gemäß dem Oberbegriff des Anspruchs 1.

Weiterhin ist aus EP 2 368 704 A2 ein Tablettenablauf bekannt mit einer darin angeordneten Weiche, die einen Führungskanal für Tabletten aufweist, wobei ein Führungsgetriebe vorgesehen ist, mit dem der Führungskanal zwischen einer ersten, einen Hauptablaufkanal des Tablettenablaufs mit einem ersten Ablaufkanal des Tablettenablaufs verbindenden Stellung und einer zweiten, den Hauptablaufkanal mit einem zweiten Ablaufkanal des Tablettenablaufs verbindenden Stellung bewegbar ist.

Ausgehend von dem erläuterten Stand der Technik liegt der Erfindung die Aufgabe zugrunde, einen Tablettenablauf und ein Verfahren der eingangs genannten Art bereitzustellen, mit denen auch bei hohen Produktionskapazitäten die Verfügbarkeit der Rundläufertablettenpresse maximiert und der Aufwand und die Gesundheitsgefährdung für Bedienpersonen minimiert werden.

Die Erfindung löst die Aufgabe durch die unabhängigen Ansprüche 1 und 9. Vorteilhafte Ausgestaltungen finden sich in den abhängigen Ansprüchen, der Beschreibung und den Figuren.

Für einen Tablettenablauf der eingangs genannten Art löst die Erfindung die Aufgabe dadurch, dass Detektionssignale des mindestens einen Sensors an der Steuereinrichtung anliegen, und dass die Steuereinrichtung dazu ausgebildet ist, bei Vorliegen eines Schaltsignals zum Bewegen der Weiche aus einer Ausgangsschaltstellung in eine Zielschaltstellung und bei Nichtvorliegen eines Detektionssignals für das Erreichen der Zielschaltstellung ein Schaltsignal zum teilweisen oder vollständigen Zurückbewegen der Weiche in die Ausgangsschaltstellung auszugeben, und anschließend ein Schaltsignal zum erneuten Bewegen der Weiche in die Zielschaltstellung auszugeben.

Für ein Verfahren der eingangs genannten Art löst die Erfindung die Aufgabe dadurch, dass wenn bei einem Bewegen der Weiche aus einer Ausgangsschaltstellung in eine Zielschaltstellung das Erreichen der Zielschaltstellung nicht detektiert wird, die Weiche teilweise oder vollständig zurück in die Ausgangsschaltstellung bewegt wird und anschließend erneut in die Zielschaltstellung bewegt wird.

Die Weiche kann zwischen einer ersten Schaltstellung, die beispielsweise eine Ausgangsschaltstellung bilden kann, und mindestens einer zweiten Schaltstellung, die beispielsweise eine Zielschaltstellung bilden kann, bewegt werden. Die erste Schaltstellung und die mindestens eine zweite Schaltstellung bzw. die Ausgangsschaltstellung und die Zielschaltstellung sind jeweils Endstellungen der Weiche bzw. einer Weichenfahne der Weiche. In diesen Endstellungen ist eine Trennung zwischen Ablaufkanälen der Weiche und damit eine Führung des Tablettenstroms in den gewünschten Tablettenausgang sichergestellt. In mindestens einer Endstellung kann die Weiche bzw. eine Weichenfahne beispielsweise an einer Innenwand eines Ablaufkanals des Tablettenablaufs anliegen. Sie kann aber auch in mindestens einer Endstellung einen Teil einer Trennwand zwischen unterschiedlichen Ablaufkanälen des Tablettenablaufs bilden.

Erfindungsgemäß ist eine Steuereinrichtung vorgesehen, die ein Schaltsignal zum Bewegen der Weiche zwischen einer ersten Schaltstellung und mindestens einer zweiten Schaltstellung ausgibt. Die Steuereinrichtung kann das Schaltsignal zum Beispiel aufgrund von Messwerten einer Sensorik der Tablettenpresse oder aufgrund von Eingaben zum Ziehen eines Musterzugs abgeben. Beispielsweise können Sensoren der Rundläufertablettenpresse unerlaubte Parameterabweichungen von hergestellten Tabletten erkennen. Diese Messwerte können an der Steuereinrichtung anliegen und die Steuereinrichtung kann entsprechend ein Schaltsignal zum Bewegen der Weiche in eine Schaltstellung ausgeben, in der die entsprechenden Tabletten einem Tablettenausgang für Schlechttabletten zugeführt werden. Auch kann die Steuereinrichtung bei Vorliegen eines Signals für das Ziehen eines Musterzugs ein Schaltsignal zum Bewegen der Weiche in eine Schaltstellung ausgeben, in der eine vorgegebene Tablettenmenge einem Tablettenausgang für einen Musterzug, also eine Stichprobenuntersuchung, zugeführt wird. Die Steuereinrichtung kann gleichzeitig die Steuereinrichtung zum Steuern des Betriebs der Rundläufertablettenpresse (Maschinensteuerung) sein. Es kann sich aber auch um eine von einer solchen Maschinensteuerung getrennte Steuereinrichtung handeln.

Weiterhin ist eine Antriebseinrichtung vorgesehen, die die Weiche abhängig von dem von der Steuereinrichtung erhaltenen Schaltsignal in die gewünschte Schaltstellung bewegt. Die Antriebseinrichtung kann zum Beispiel einen Elektromotor umfassen, der die Weiche zwischen der ersten und der mindestens einen zweiten Schaltstellung bewegt, zum Beispiel eine Weichenfahne verschwenkt. Denkbar wären aber zum Beispiel auch elektromagnetische Antriebe. In an sich bekannter Weise ist weiterhin mindestens ein Sensor vorgesehen, der eine oder mehrere, insbesondere sämtliche Endstellungen der Weiche, also die erste und/oder die mindestens eine zweite Schaltstellung bzw. die Ausgangsschaltstellung und die Zielschaltstellung detektiert. Es können hierzu mehrere Sensoren vorgesehen sein. Das Detektieren der Endstellungen kann dabei direkt oder indirekt erfolgen. Beispielsweise kann der Sensor oder können die die Sensoren auch aus anderen Messwerten auf das Erreichen bzw. Nichterreichen der jeweiligen Endstellung schließen. Als Sensoren kommen zum Beispiel Induktionssensoren oder Mikroschalter infrage, die das Erreichen bzw. Nichterreichen der jeweils vorgegebenen Endstellung erkennen. Es kann sich aber selbstverständlich auch um andere Sensoren handeln. Weiter beispielhaft genannt sei ein Winkelsensor, der die Winkellage der Weiche, beispielsweise einer Weichenfahne, erfasst und so alle Endstellungen und vorzugsweise auch beliebige Zwischenstellungen der Weiche erfassen kann.

Bei einem Bewegen der Weiche aus einer Ausgangsschaltstellung, zum Beispiel der ersten Schaltstellung, in eine Zielschaltstellung, zum Beispiel der mindestens einen zweiten Schaltstellung, detektiert der mindestens eine Sensor, ob die Zielschaltstellung erreicht wurde oder nicht. Die Messsignale des mindestens einen Sensors liegen an der Steuereinrichtung an. Wird trotz Schaltsignals der Steuereinrichtung zum Bewegen der Weiche aus einer Ausgangsschaltstellung in die Zielschaltstellung das Erreichen der Zielschaltstellung durch den mindestens einen Sensor nicht detektiert, gibt die Steuereinrichtung ein Schaltsignal an die Antriebseinrichtung zum teilweisen oder auch vollständigen Zurückbewegen der Weiche in Richtung der Ausgangsschaltstellung ab. Die Antriebseinrichtung stellt die Weiche entsprechend unter Umkehrung ihrer Bewegungsrichtung teilweise oder vollständig zurück in Richtung der Ausgangsschaltstellung. Anschließend gibt die Steuereinrichtung ein Schaltsignal an die Antriebseinrichtung zum (wieder) Zurückbewegen der Weiche in die Zielschaltstellung unter erneuter Umkehrung der Bewegungsrichtung ab. Die Antriebseinrichtung stellt die Weiche entsprechend wieder zurück in die Zielschaltstellung. Es kann beispielsweise ein Grenzzeitraum nach dem Ausgeben des Schaltsignals vorgegeben werden, innerhalb dem das Detektionssignal des mindestens einen Sensors für das Erreichen der Zielschaltstellung vorliegen muss. Wird dieser Grenzzeitraum überschritten, ohne dass das Detektionssignal für das Erreichen der Zielschaltstellung vorliegt, wird die Weiche teilweise zurückbewegt. Dieser Grenzzeitraum kann beispielsweise geringfügig größer gewählt werden als der für das Schalten der Weiche aus der Ausgangsschaltstellung in die Zielschaltstellung erforderliche Zeitraum.

Der Erfindung liegt der Gedanke zugrunde, dass bei Nichtvorliegen eines Detektionssignals für das Erreichen der Zielschaltstellung eine Tablette zwischen der Weiche und einer Wand des Tablettenablaufs eingeklemmt ist. Um diese eingeklemmte Tablette für einen Ablauf freizugeben, wird die Weiche zurück in Richtung ihrer Ausgangsschaltstellung bewegt. Die zwischen der Weiche, beispielsweise einer Weichenfahne, und einer gegenüberliegenden Wand des Tablettenablaufs eingeklemmte Tablette wird somit für ein Abfließen freigegeben und die Weiche kann danach die Zielschaltstellung sicher einnehmen.

Die Erfindung geht somit davon aus, dass es weiterhin zu einem Einklemmen von Tabletten kommen kann. Es werden jedoch Maßnahmen ergriffen, um das Vorliegen eines solchen Einklemmens sofort und automatisch durch die Steuereinrichtung zu beheben. Insbesondere wird, wenn die vorgegebene Endstellung der Weiche nicht erreicht wird, diese Information genutzt, um die Bewegungsrichtung der Weiche umzukehren. Durch das Umkehren der Bewegungsrichtung werden eingeklemmte Tabletten freigegeben und können den Weichenbereich verlassen. Anschließend kann die Endstellung zuverlässig angefahren werden.

Indem erfindungsgemäß ein etwaiges Einklemmen von Tabletten direkt und automatisch behoben wird, wird ein Tablettenstau in dem Tablettenablauf mit den erläuterten negativen Folgen sicher vermieden. Eine Weichenfehlfunktion, die in der Praxis eine der häufigsten Ursachen für den Stillstand einer Rundläufertablettenpresse ist, wird vermieden oder zumindest derart entschärft, dass Folgeprobleme, wie ein Tablettenstau und die hiermit verbundenen Konsequenzen, ausbleiben.

Indem ein Tablettenstau vermieden wird, wird die Zuverlässigkeit und Verfügbarkeit der Rundläufertablettenpresse erhöht. Bedienpersonen werden durch das automatisierte Beheben der Weichenfehlfunktion vor toxischen Produkten und einer damit verbundenen Gesundheitsgefährdung geschützt. Manueller Aufwand zum Beheben einer Weichenfehlfunktion wird vermieden. Indem darüber hinaus ein Stillstand der Rundläufertablettenpresse und ein anschließender Anfahrvorgang vermieden werden können, wird entstehender Ausschuss im Vergleich zum Stand der Technik erheblich reduziert. Darüber hinaus kann eine erhöhte Tablettenqualität durch einen geringeren mechanischen Stress der Tabletten erreicht werden.

Das erläuterte Verfahren zum Beheben eines Einklemmens von Tabletten kann solange wiederholt werden bis das Erreichen der Zielschaltstellung durch die Weiche detektiert wird. Die erfindungsgemäße Steuereinrichtung kann entsprechend dazu ausgebildet sein.

Der Tablettenablauf kann wie an sich bekannt mindestens einen Ablaufkanal aufweisen. Insbesondere kann der Tablettenablauf mehrere Ablaufkanäle aufweisen, zwischen denen die Weiche den Tablettenstrom umlenken kann. Jeder Ablaufkanal kann zu einem der Tablettenausgänge führen. Wie bereits erläutert, können die Tablettenausgänge beispielsweise ein Ausgang für Schlechttabletten, für Guttabletten oder zu einer Messeinrichtung für einen Musterzug führen.

Die Weiche kann mindestens eine in dem mindestens einen Ablaufkanal des Tablettenablaufs schwenkbar zwischen der ersten Schaltstellung und der mindestens einen zweiten Schaltstellung gelagerte Weichenfahne umfassen. Eine solche Weichenfahne ist ein länglicher Körper, der vorzugsweise im Bereich seines einen Endes schwenkbar an dem Ablaufkanal gelagert ist. Das gegenüberliegende freie Ende der Weichenfahne führt dann die maximale Schwenkbewegung aus. Das freie Ende der Weichenfahne kann in Ablaufrichtung der Tabletten gesehen vor der Schwenklagerung angeordnet sein. Die Weichenfahne kann zum Beispiel aus einem Metallwerkstoff bestehen. Dies ist an sich bekannt. Selbstverständlich kann die Weiche auch mehrere solcher, in Strömungsrichtung der Tabletten hintereinander angeordnete Weichenfahnen umfassen, um beispielsweise zwischen drei Ablaufkanälen schalten zu können.

Gemäß einer weiteren Ausgestaltung kann mindestens ein Ablaufkanal mindestens einen sich in Ablaufrichtung der Tabletten erweiternden, vorzugsweise sich stufenartig erweiternden, Abschnitt aufweisen. Der mindestens eine sich erweiternde Abschnitt kann sich gemäß einer weiteren Ausgestaltung in Ablaufrichtung der Tabletten vor dem, vorzugsweise unmittelbar vor dem, von der Weichenfahne, beispielsweise ihrem freien Ende, bei ihrer Schwenkbewegung überstrichenen Bereich befinden. Weiterhin ist es vorteilhaft, wenn an gegenüberliegenden Wänden des Ablaufkanals jeweils mindestens ein sich erweiternder, vorzugsweise sich stufenartig erweiternder, Abschnitt vorgesehen ist. Die Erweiterung des mindestens einen sich erweiternden Abschnitts kann nach einer weiteren Ausgestaltung eine Breite von mindestens 10 mm, vorzugsweise mindestens 19 mm, weiter vorzugsweise mindestens 25 mm aufweisen. Die Breite wird in einer Richtung senkrecht zur Strömungsrichtung der Tabletten bzw. der Längsachse des Ablaufkanals gemessen. Die Breite ist dabei bevorzugt mindestens so breit wie die größte durch den Ablaufkanal zu fördernde Tablette.

Die vorgenannte vorzugsweise stufenartige Erweiterung bildet eine Schaltbucht. Insbesondere vergrößert sich hier die Breite des Ablaufkanals. Die Erweiterung kann sich im Bereich einer oder sämtlicher Zielschaltstellungen der Weiche befinden. Die Erweiterung ist derart ausgeführt, dass ein Einklemmen von Tabletten zwischen der Weiche und einer gegenüberliegenden Wand des Ablaufkanals den übrigen Tablettenstrom nicht behindert. Sofern die Weiche ihre Zielschaltstellung nicht erreicht, weil durch die Weiche eine Tablette eingeklemmt wird, befindet sich die eingeklemmte Tablette bereits innerhalb des erweiterten Abschnitts des Ablaufkanals. Damit wird sichergestellt, dass das Nichterreichen der Zielschaltstellung wegen einer eingeklemmten Tablette für den übrigen Tablettenstrom kein Hindernis bildet. Ein Tablettenstau wird besonders sicher verhindert. Der sich erweiternde Abschnitt kann insbesondere derart ausgestaltet sein, dass bei Nichterreichen der Zielschaltstellung durch die Weiche bei durch die Weiche eingeklemmter Tablette die Weiche im Wesentlichen nicht (nach innen) über den in Ablaufrichtung der Tabletten unmittelbar vor dem sich erweiternden Abschnitt befindlichen Führungsabschnitt des Ablaufkanals vorsteht. Dieser Führungsabschnitt kann durch eine Wand des Ablaufkanals oder bei mehreren hintereinander angeordneten Weichen bzw. Weichenfahnen auch durch eine in Ablaufrichtung vorgeordnete Weiche bzw. Weichenfahne gebildet sein.

Die Erfindung betrifft auch eine Rundläufertablettenpresse, umfassend einen mittels eines Drehantriebs drehbaren Rotor, wobei der Rotor eine obere Stempelführung für obere Stempel der Rundläufertablettenpresse und eine untere Stempelführung für untere Stempel der Rundläufertablettenpresse sowie eine zwischen den Stempelführungen angeordnete Matrizenschreibe aufweist, wobei die Stempel mit Kavitäten der Matrizenscheibe zusammenwirken, weiter umfassend eine Fülleinrichtung, durch die zu verpressendes Füllmaterial in die Kavitäten der GeäMatrizenscheibe gefüllt wird, weiter umfassend mindestens eine obere Druckeinrichtung und mindestens eine untere Druckeinrichtung, die im Betrieb mit den oberen Stempeln und mit den unteren Stempeln zum Verpressen des Füllmaterials in den Kavitäten der Matrizenscheibe zu Tabletten zusammenwirken, weiter umfassend eine Auswurfeinrichtung, in der in den Kavitäten erzeugte Tabletten ausgeworfen werden, und umfassend einen erfindungsgemäßen Tablettenablauf, dem die ausgeworfenen Tabletten zugeführt werden. In dem Tablettenablauf ist mindestens eine Weiche angeordnet.

Das erfindungsgemäße Verfahren kann mit einem erfindungsgemäßen Tablettenablauf oder einer erfindungsgemäßen Rundläufertablettenpresse durchgeführt werden. Entsprechend kann der erfindungsgemäße Tablettenablauf oder die erfindungsgemäße Rundläufertablettenpresse zur Durchführung des erfindungsgemäßen Verfahrens ausgebildet sein.

Ein Ausführungsbeispiel der Erfindung wird nachfolgend anhand von Figuren näher erläutert. Es zeigen schematisch:
- Figur 1: eine erfindungsgemäße Rundläuferpresse in einer abgewickelten Darstellung des Rotors,
- Figur 2: eine Weiche der in Figur 1 gezeigten Rundläufertablettenpresse in einer ersten Betriebsstellung,
- Figur 3: die Weiche aus Figur 2 in einer zweiten Betriebsstellung,
- Figur 4: die Weiche aus Figur 2 in einer dritten Betriebsstellung, und
- Figur 5: die Weiche aus Figur 2 in einer vierten Betriebsstellung.

Soweit nichts anderes angegeben ist, bezeichnen in den Figuren gleiche Bezugszeichen gleiche Gegenstände.

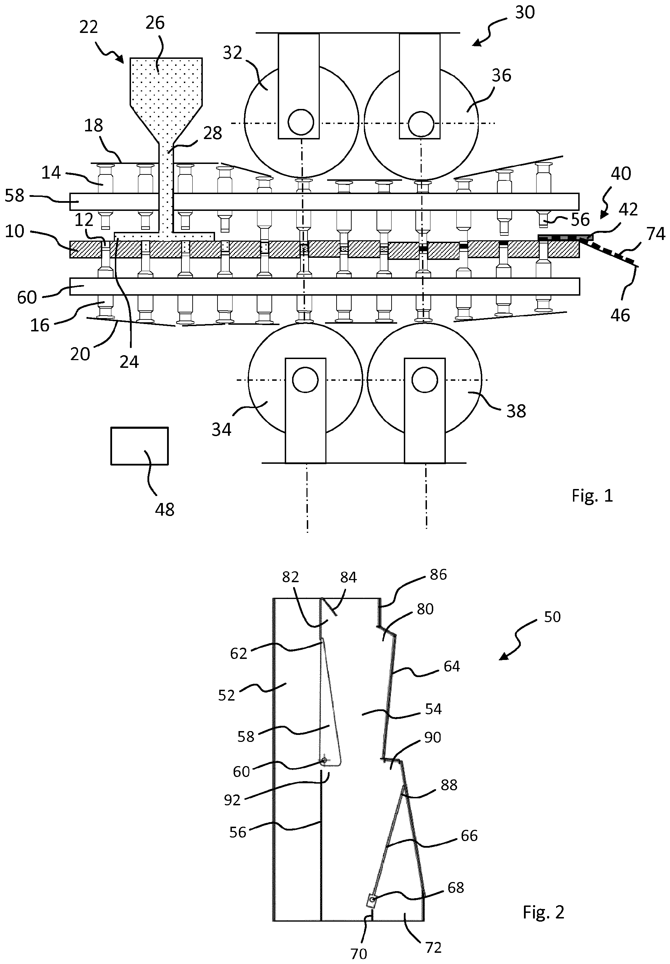

Die in Figur 1 gezeigte Rundläufertablettenpresse umfasst einen durch einen nicht näher dargestellten Drehantrieb drehend angetriebenen Rotor mit einer Matrizenscheibe 10, die eine Mehrzahl von Kavitäten 12 aufweist. Die Kavitäten 12 können beispielsweise durch Bohrungen der Matrizenscheibe 10 gebildet sein. Weiter umfasst der Rotor eine Mehrzahl von oberen Stempeln 14 und unteren Stempeln 16, die mit der Matrizenscheibe 10 synchron umlaufen. Jeweils ein Paar aus oberem Stempel 14 und unterem Stempel 16 ist dabei einer Kavität 12 zugeordnet. Die axiale Bewegung der oberen Stempel 14 und unteren Stempel 16 im Zuge der Drehung des Rotors wird durch obere Steuerkurvenelemente 18 und untere Steuerkurvenelemente 20 gesteuert. Die Rundläufertablettenpresse umfasst weiterhin eine Fülleinrichtung 22, die eine Füllkammer 24 aufweist. Die Fülleinrichtung 22 umfasst darüber hinaus ein trichterförmiges Füllmaterialreservoir 26, das über einen Zuführabschnitt 28 mit der Füllkammer 24 in Verbindung steht. Auf diese Weise gelangt in dem vorliegenden Beispiel pulverförmiges Füllmaterial aus dem Füllmaterialreservoir 26 über den Zuführabschnitt 28 schwerkraftbedingt in die Füllkammer 24 und aus dieser über eine an der Unterseite der Füllkammer 24 vorgesehene Befüllöffnung wiederum schwerkraftbedingt in die Kavitäten 12 der Matrizenscheibe 10.

Außerdem umfasst die Rundläufertablettenpresse eine Druckeinrichtung 30. Die Druckeinrichtung 30 besitzt eine Vordruckeinrichtung mit einer oberen Vordruckrolle 32 und einer unteren Vordruckrolle 34 sowie eine Hauptdruckeinrichtung mit einer oberen Hauptdruckrolle 36 und einer unteren Hauptdruckrolle 38. Darüber hinaus umfasst die Rundläufertablettenpresse eine Auswurfeinrichtung 40, vorliegend mit einem Abstreifer 42, der die in der Rundläuferpresse hergestellten Tabletten 74 einem Tablettenablauf 46 zuführt.

Eine Steuereinrichtung zum Betrieb der Rundläuferpresse ist bei dem Bezugszeichen 48 gezeigt. Die Steuereinrichtung 48 ist über nicht näher dargestellte Leitungen unter anderem mit dem Drehantrieb des Rotors verbunden.

In dem Tablettenablauf 46 befindet sich eine in den Figuren 2 bis 5 dargestellte Weiche 50. Die Weiche 50 weist einen ersten Ablaufkanal 52 und einen zweiten Ablaufkanal 54 auf. Der erste und zweite Ablaufkanal 52, 54 sind durch eine Trennwand 56 voneinander getrennt. In der Trennwand 56 befindet sich eine erste Weichenfahne 58, die um eine erste Schwenkachse 60 schwenkbar zwischen der in Figur 2 gezeigten ersten Schaltstellung und einer nicht gezeigten zweiten Schaltstellung, in der das freie Ende 62 der Weichenfahne 58 an der gegenüberliegenden Wand 64 des zweiten Ablaufkanals 54 anliegt, gelagert ist. Tabletten fließen in den Figuren 2 bis 5 von oben nach unten durch die Weiche 50.

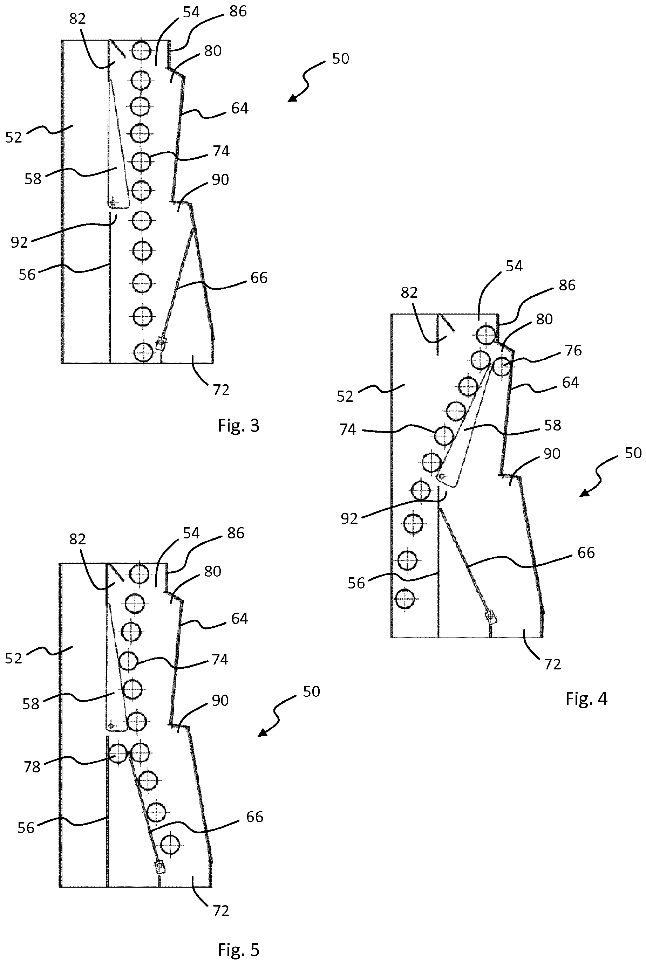

Stromab der ersten Weichenfahne 58 ist in dem zweiten Ablaufkanal 54 eine zweite Weichenfahne 66 um eine zweite Schwenkachse 68 schwenkbar gelagert zwischen einer in Figur 2 gezeigten ersten Schaltstellung und einer in Figur 4 gezeigten zweiten Schaltstellung. Stromab der zweiten Weichenfahne 66 ist eine Trennwand 70 zu erkennen, die einen dritten Ablaufkanal 72 definiert. Zum Verschwenken der ersten Weichenfahne 58 und der zweiten Weichenfahne 66 zwischen ihrer jeweiligen ersten und zweiten Schaltstellung sind Antriebe einer Antriebseinrichtung vorgesehen, beispielsweise elektromotorische oder elektromagnetische Antriebe. Außerdem sind nicht näher dargestellte, an sich bekannte Sensoren vorgesehen, die das Erreichen der jeweiligen Endstellungen der Weichenfahnen 58, 66, also der jeweiligen ersten Schaltstellung und der jeweiligen zweiten Schaltstellung, detektieren. Die Detektionssignale der Sensoren liegen in dem gezeigten Beispiel ebenfalls an der Steuereinrichtung 48 an.

In den Figuren 3 bis 5 sind für unterschiedliche Betriebszustände der Weiche 50 durch die Weiche 50 ablaufende Tabletten 74 gezeigt. Wie in Figur 3 zu erkennen, laufen die Tabletten 74 bei in der ersten Schaltstellung befindlicher ersten Weichenfahne 58 und zweiten Weichenfahne 66 durch den zweiten Ablaufkanal 54 hindurch ab. Wird dagegen die erste Weichenfahne 58 in Richtung ihrer zweiten Schaltstellung bewegt, wie in Figur 4 gezeigt, wird der Tablettenstrom 74 aus dem zweiten Ablaufkanal 54 in den ersten Ablaufkanal 52 abgelenkt. Nur wenn sich die erste Weichenfahne 58 in ihrer ersten Schaltstellung befindet, gelangt der Tablettenstrom 74 zu der zweiten Weichenfahne 66. Diese lenkt den Tablettenstrom 74 dann je nach ihrer Schaltstellung in den dritten Ablaufkanal 72 ab (siehe Figur 5), oder verschließt den Zugang zu dem dritten Ablaufkanal 72, so dass der Tablettenstrom 74 weiter durch den zweiten Ablaufkanal 54 abfließen kann.

Der zweite Ablaufkanal 54 kann zum Beispiel zu einem Tablettenausgang für Guttabletten führen. Der dritte Ablaufkanal 72 kann zum Beispiel zu einem Tablettenausgang für einen Musterzug führen. Der erste Ablaufkanal 52 kann zum Beispiel zu einem Tablettenausgang für Schlechttabletten führen.

In Figur 4 ist eine zwischen der ersten Weichenfahne 58 und der gegenüberliegenden Wand 64 eingeklemmte Tablette 76 zu erkennen. Dadurch kann die erste Weichenfahne 58 ihre zweite Schaltstellung nicht erreichen. Das Nichterreichen der zweiten Schaltstellung wird durch den jeweiligen Sensor detektiert und die Steuereinrichtung gibt auf dieser Grundlage ein Schaltsignal an den Antrieb der ersten Weichenfahne 58 aus, so dass dieser die Weichenfahne 58 teilweise zurück in Richtung der ersten Schaltstellung schwenkt, in Figur 4 also entgegen dem Uhrzeigersinn. Dadurch wird die eingeklemmte Tablette 76 freigegeben und diese kann weiter abfließen. Hierfür ist ein geringfügiges Zurückbewegen der Weichenfahne 58 ausreichend, so dass die Tablette 76 freigegeben wird. Anschließend steuert die Steuereinrichtung den Antrieb durch Ausgabe eines entsprechenden Schaltsignals wieder derart an, dass dieser die erste Weichenfahne 58 zurück in die zweite Schaltstellung bewegt, die diese nun aufgrund der freigegebenen Tablette 76 problemlos erreichen kann.

In Figur 5 ist der Fall dargestellt, bei dem die zweite Weichenfahne 66 beim Bewegen aus ihrer ersten Schaltstellung in ihre zweite Schaltstellung eine Tablette 78 zwischen sich und der gegenüberliegenden Trennwand 56 einklemmt. Wiederum kann die zweite Weichenfahne 66 ihre zweite Schaltstellung nicht erreichen. Aufgrund eines diesbezüglichen Detektionssignals des zugeordneten Sensors bzw. dessen Ausbleibens gibt die Steuereinrichtung 48 ein Schaltsignal an den Antrieb der zweiten Weichenfahne 66 aus, so dass dieser die zweite Weichenfahne 66 teilweise in Richtung ihrer ersten Schaltstellung zurück bewegt, in Figur 5 also im Uhrzeigersinn verschwenkt. Dadurch wird die eingeklemmte Tablette 78 freigegeben und diese kann abfließen. Wiederum ist ein geringfügiges Zurückbewegen der Weichenfahne 66 ausreichend, so dass die Tablette 78 freigegeben wird.

Anschließend wird die zweite Weichenfahne 66 durch ein entsprechendes Schaltsignal der Steuereinrichtung 48 zurück in ihre zweite Schaltstellung bewegt, wobei sie diese nun vollständig erreichen kann.

In den Figuren 2 bis 5 ist außerdem zu erkennen, dass sich der zweite Ablaufkanal 54 unmittelbar vor dem von der ersten Weichenfahne 58 im Zuge ihrer Schwenkbewegung überstrichenen Bereich, insbesondere dem durch ihr freies Ende 62 überstrichenen Bereich, beidseitig in der Breite erweitert. Die sich erweiternden Abschnitte sind bei den Bezugszeichen 80 und 82 gezeigt. Dabei ist erkennbar, dass der sich erweiternde Abschnitt 82 dadurch gebildet ist, dass eine sich stromauf aufgrund eines Wandvorsprungs 84 befindende Verengung endet. Die sich im gezeigten Beispiel stufenartig erweiternden Abschnitte 80, 82 entsprechen in dem gezeigten Beispiel jeweils im Wesentlichen der Breite der durch die Weiche 50 abzuleitenden Tabletten 74, 76, 78. Wie in Figur 4 beispielhaft für den sich erweiternden Abschnitt 80 gezeigt, führen diese sich erweiternden Abschnitte 80, 82 dazu, dass der Tablettenstrom 74 durch das Einklemmen einer Tablette 76 und das dadurch verursachte Nichterreichen der jeweiligen Endstellung durch die jeweilige Weichenfahne nicht beeinträchtigt wird. So befindet sich die eingeklemmte Tablette 76 bereits innerhalb des erweiterten Abschnitts 80. Das freie Ende 62 der ersten Weichenfahne 58 steht im Wesentlichen nicht nach innen über den unmittelbar stromauf des erweiterten Abschnitts 80 befindlichen Wandabschnitt 86 hervor. Der an der gegenüberliegenden Seite vorgesehene erweiterte Abschnitt 82 bietet insoweit die gleiche Funktion.

Auch unmittelbar vor dem durch das freie Ende 88 der zweiten Weichenfahne 66 bei ihrer Schwenkbewegung überstrichenen Bereich befinden sich in dem zweiten Ablaufkanal 54 auf gegenüberliegenden Seiten erweiterte Abschnitte 90, 92. Der erweiterte Abschnitt 92 wird dabei durch das Ende des eine Verengung des zweiten Ablaufkanals 54 bildenden, in Figur 2 unteren Endes der ersten Weichenfahne 58 gebildet. In gleicher Weise, wie oben zu der ersten Weichenfahne 58 erläutert, führen auch die erweiterten Abschnitte 90, 92 dazu, dass eine durch die zweite Weichenfahne 66 eingeklemmte Tablette 78 und das dadurch nicht mögliche Erreichen der jeweiligen Endstellung durch die zweite Weichenfahne 66 den Tablettenstrom 74 nicht beeinträchtigt. Beispielhaft ist dies in Figur 5 für die Erweiterung 92 gezeigt. Wiederum entspricht die Erweiterung 90, 92 im Wesentlichen in ihrer Breite dem Durchmesser der durch die Weiche 50 abzuleitenden Tabletten 74, 76, 78.

### Bezugszeichenliste

- 10: Matrizenscheibe
- 12: Kavitäten
- 14: Obere Stempel
- 16: Untere Stempel
- 18: Obere Steuerkurvenelemente
- 20: Untere Steuerkurvenelemente
- 22: Fülleinrichtung
- 24: Füllkammer
- 26: Füllmaterialreservoir
- 28: Zuführabschnitt
- 30: Druckeinrichtung
- 32: Obere Vordruckrolle
- 34: Untere Vordruckrolle
- 36: Obere Hauptdruckrolle
- 38: Untere Hauptdruckrolle
- 40: Auswurfeinrichtung
- 42: Abstreifer
- 46: Tablettenablauf
- 48: Steuereinrichtung
- 50: Weiche
- 52: Erster Ablaufkanal
- 54: Zweiter Ablaufkanal
- 56: Trennwand
- 58: Erste Weichenfahne
- 60: Erste Schwenkachse
- 62: Freies Ende
- 64: Wand
- 66: Zweite Weichenfahne
- 68: Zweite Schwenkachse
- 70: Trennwand
- 72: Dritter Ablaufkanal
- 74: Tabletten
- 76: Tablette
- 78: Tablette
- 80: Erweiterter Abschnitt
- 82: Erweiterter Abschnitt
- 84: Wandvorsprung
- 86: Wandabschnitt
- 88: Freies Ende
- 90: Erweiterter Abschnitt
- 92: Erweiterter Abschnitt

## Patentansprüche

1. Tablettenablauf einer Tablettenpresse, insbesondere einer Rundläufertablettenpresse, mit einer in dem Tablettenablauf (46) angeordneten Weiche (50), wobei eine Steuereinrichtung (48) vorgesehen ist, und wobei eine Antriebseinrichtung vorgesehen ist, die die Weiche (50) abhängig von einem Schaltsignal der Steuereinrichtung (48) zwischen einer ersten Schaltstellung, in der Tabletten (74, 76, 78) einem ersten Tablettenausgang zugeleitet werden, und mindestens einer zweiten Schaltstellung, in der Tabletten (74, 76, 78) mindestens einem zweiten Tablettenausgang zugeleitet werden, bewegt, wobei mindestens ein Sensor vorgesehen ist zum Detektieren des Erreichens der ersten Schaltstellung und/oder der mindestens einen zweiten Schaltstellung durch die Weiche (50), **dadurch gekennzeichnet, dass** Detektionssignale des mindestens einen Sensors an der Steuereinrichtung (48) anliegen, und dass die Steuereinrichtung (48) dazu ausgebildet ist, bei Vorliegen eines Schaltsignals zum Bewegen der Weiche (50) aus einer Ausgangsschaltstellung in eine Zielschaltstellung und bei Nichtvorliegen eines Detektionssignals für das Erreichen der Zielschaltstellung ein Schaltsignal zum teilweisen oder vollständigen Zurückbewegen der Weiche (50) in die Ausgangsschaltstellung auszugeben, und anschließend ein Schaltsignal zum erneuten Bewegen der Weiche (50) in die Zielschaltstellung auszugeben.

2. Tablettenablauf nach Anspruch 1, **dadurch gekennzeichnet, dass** der Tablettenablauf (46) mindestens einen Ablaufkanal (52, 54, 72) aufweist.

3. Tablettenablauf nach Anspruch 2, **dadurch gekennzeichnet, dass** die Weiche (50) mindestens eine in dem mindestens einen Ablaufkanal (52, 54, 72) des Tablettenablaufs (46) schwenkbar zwischen der ersten Schaltstellung und der mindestens einen zweiten Schaltstellung gelagerte Weichenfahne (58, 66) umfasst.

4. Tablettenablauf nach einem der Ansprüche 2 oder 3, **dadurch gekennzeichnet, dass** mindestens ein Ablaufkanal (52, 54, 72) mindestens einen sich in Ablaufrichtung der Tabletten (74, 76, 78) erweiternden, vorzugsweise sich stufenartig erweiternden, Abschnitt (80, 82, 90, 92) aufweist.

5. Tablettenablauf nach Anspruch 3 und 4, **dadurch gekennzeichnet, dass** sich der mindestens eine sich erweiternde Abschnitt (80, 82, 90, 92) in Ablaufrichtung der Tabletten (74, 76, 78) vor dem, vorzugsweise unmittelbar vor dem, von der Weichenfahne (58, 66) bei ihrer Schwenkbewegung überstrichenen Bereich befindet.

6. Tablettenablauf nach einem der Ansprüche 4 oder 5, **dadurch gekennzeichnet, dass** an gegenüberliegenden Wänden des Ablaufkanals (52, 54, 72) jeweils mindestens ein sich erweiternder, vorzugsweise sich stufenartig erweiternder, Abschnitt (80, 82, 90, 92) vorgesehen ist.

7. Tablettenablauf nach einem der Ansprüche 4 bis 6, **dadurch gekennzeichnet, dass** die Erweiterung des mindestens einen sich erweiternden Abschnitts (80, 82, 90, 92) eine Breite von mindestens 10 mm, vorzugsweise mindestens 19 mm, weiter vorzugsweise mindestens 25 mm aufweist.

8. Rundläufertablettenpresse, umfassend einen mittels eines Drehantriebs drehbaren Rotor, wobei der Rotor eine obere Stempelführung für obere Stempel (14) der Rundläufertablettenpresse und eine untere Stempelführung für untere Stempel (16) der Rundläufertablettenpresse sowie eine zwischen den Stempelführungen angeordnete Matrizenschreibe (10) aufweist, wobei die Stempel (14, 16) mit Kavitäten (12) der Matrizenscheibe (10) zusammenwirken, weiter umfassend eine Fülleinrichtung (22), durch die zu verpressendes Füllmaterial in die Kavitäten (12) der Matrizenscheibe (10) gefüllt wird, weiter umfassend mindestens eine obere Druckeinrichtung (30) und mindestens eine untere Druckeinrichtung (30), die im Betrieb mit den oberen Stempeln (14) und mit den unteren Stempeln (16) zum Verpressen des Füllmaterials in den Kavitäten (12) der Matrizenscheibe (10) zu Tabletten (74, 76, 78) zusammenwirken, weiter umfassend eine Auswurfeinrichtung (40), in der in den Kavitäten (12) erzeugte Tabletten ausgeworfen werden, **dadurch gekennzeichnet, dass** die Rundläufertablettenpresse einen Tablettenablauf (46) nach einem der vorhergehenden Ansprüche umfasst, dem die ausgeworfenen Tabletten (74, 76, 78) zugeführt werden.

9. Verfahren zum Betätigen einer Weiche (50) eines Tablettenablaufs (46) einer Tablettenpresse, insbesondere einer Rundläufertablettenpresse, bei dem die Weiche (50) zwischen einer ersten Schaltstellung, in der Tabletten (74, 76, 78) einem ersten Tablettenausgang zugeleitet werden, und mindestens einer zweiten Schaltstellung, in der Tabletten (74, 76, 78) mindestens einem zweiten Tablettenausgang zugeleitet werden, bewegt wird, wobei mittels mindestens eines Sensors das Erreichen der ersten Schaltstellung und/oder der mindestens einen zweiten Schaltstellung durch die Weiche (50) detektiert wird, **dadurch gekennzeichnet, dass** wenn bei einem Bewegen der Weiche (50) aus einer Ausgangsschaltstellung in eine Zielschaltstellung das Erreichen der Zielschaltstellung nicht detektiert wird, die Weiche (50) teilweise oder vollständig zurück in die Ausgangsschaltstellung bewegt wird und anschließend erneut in die Zielschaltstellung bewegt wird.

10. Verfahren nach Anspruch 9, **dadurch gekennzeichnet, dass** das Verfahren so lange wiederholt wird bis das Erreichen der Zielschaltstellung durch die Weiche (50) detektiert wird.

11. Verfahren nach einem der Ansprüche 9 oder 10, **dadurch gekennzeichnet, dass** es mit einem Tablettenablauf (46) nach einem der Ansprüche 1 bis 7 oder einer Rundläufertablettenpresse nach Anspruch 8 durchgeführt wird.

## Claims

1. A tablet discharge of a tablet press, in particular of a rotary tablet press, having a switch (50) arranged in the tablet discharge (46), wherein a control apparatus (48) is provided, and wherein a drive apparatus is provided that moves the switch (50), depending on a switching signal from the control apparatus (48), between a first switching position in which tablets (74, 76, 78) are fed to a first tablet outlet, and at least one second switching position in which tablets (74, 76, 78) are fed to at least one second tablet outlet, wherein at least one sensor is provided for detecting the reaching of the first switching position and/or the at least one second switching position by the switch (50), **characterized in that** detection signals from the at least one sensor are applied to the control apparatus (48), and the control apparatus (48) is configured, when there is a switching signal, to move the switch (50) out of an initial switching position into a target switching position and, when there is no detection signal for reaching the target switching position, to output a switching signal to move the switch (50) back partially or completely into the target switching position, and to subsequently output a switching signal to move the switch (50) again into the target switching position.

2. The tablet discharge according to Claim 1, **characterized in that** the tablet discharge (46) has at least one discharge channel (52, 54, 72).

3. The tablet discharge according to Claim 2, **characterized in that** the switch (50) comprises at least one switch flap (58, 66) pivotably mounted in the at least one discharge channel (52, 54, 72) of the tablet discharge (46) between the first switching position and the at least one second switching position.

4. The tablet discharge according to any one of Claims 2 or 3, **characterized in that** at least one discharge channel (52, 54, 72) has at least one section (80, 82, 90, 92) that expands in the discharge direction of the tablets (74, 76, 78), preferably that expands in steps.

5. The tablet discharge according to Claim 3 and 4, **characterized in that** the at least one expanding section (80, 82, 90, 92) is located in the discharge direction of the tablets (74, 76, 78) before the, preferably directly before the, region swept by the switch flap (58, 66) during its pivoting movement.

6. The tablet discharge according to any one of Claims 4 or 5, **characterized in that** at least one section (80, 82, 90, 92) that expands, preferably expands in steps, is in each case provided on opposing walls of the discharge channel (52, 54, 72).

7. The tablet discharge according to any one of Claims 4 to 6, **characterized in that** the expansion of the at least one expanding section (80, 82, 90, 92) has a width of at least 10 mm, preferably at least 19 mm, and more preferably at least 25 mm.

8. A rotary tablet press comprising a rotor that can be rotated by means of a rotary drive, wherein the rotor has an upper punch guide for upper punches (14) of the rotary tablet press and a lower punch guide for lower punches (16) of the rotary tablet press, as well as a die plate (10) arranged between the punch guides, wherein the punches (14, 16) interact with cavities (12) in the die plate (10), further comprising a filling apparatus (22), by means of which the filling material to be pressed is filled into the cavities (12) in the die plate (10), further comprising at least one upper pressing apparatus (30) and at least one lower pressing apparatus (30) that, during operation, interact with the upper punches (14) and with the lower punches (16) in order to press the filling material into tablets (74, 76, 78) in the cavities (12) in the die plate (10), further comprising an ejection apparatus (40) in which tablets generated in the cavities (12) are ejected, **characterized in that** the rotary tablet press comprises a tablet discharge (46) according to any one of the preceding claims, which is fed the ejected tablets (74, 76, 78).

9. A method for actuating a switch (50) of a tablet discharge (46) of a tablet press, in particular of a rotary tablet press, in which the switch (50) is moved between a first switching position in which tablets (74, 76, 78) are fed to a first tablet outlet, and at least one second switching position in which tablets (74, 76, 78) are fed to at least one second tablet outlet, wherein the reaching of the first switching position and/or of the at least one second switching position by the switch (50) is detected by means of at least one sensor, **characterized in that** if, during a movement of the switch (50) from an initial switching position into a target switching position the reaching of the target switching position is not detected, the switch (50) is moved partially or completely back into the initial switching position and subsequently moved again into the target switching position.

10. The method according to Claim 9, **characterized in that** the method is repeated until the reaching of the target switching position by the switch (50) is detected.

11. The method according to any one of Claims 9 or 10, **characterized in that** it is performed with a tablet discharge (46) according to any one of Claims 1 to 7 or a rotary tablet press according to Claim 8.

## Revendications

1. Sortie de comprimés d'une presse à comprimés, en particulier d'une presse à comprimés rotative, avec un aiguillage (50) disposé dans la sortie de comprimés (46), dans laquelle il est prévu un dispositif de commande (48), et dans laquelle il est prévu un dispositif d'entraînement destiné à déplacer l'aiguillage (50) en fonction d'un signal de commutation du dispositif de commande (48), entre une première position de commutation, dans laquelle des comprimés (74, 76, 78) sont alimentés vers une première sortie de comprimés, et au moins une deuxième position de commutation, dans laquelle des comprimés (74, 76, 78) sont alimentés vers au moins une deuxième sortie de comprimés, dans laquelle il est prévu au moins un capteur destiné à détecter lorsque l'aiguillage (50) atteint la première position de commutation et/ou l'au moins une deuxième position de commutation, **caractérisée en ce que** des signaux de détection de l'au moins un capteur sont appliqués au dispositif de commande (48), et **en ce que** le dispositif de commande (48), en présence d'un signal de commutation pour le déplacement de l'aiguillage (50) à partir d'une position de commutation de départ vers une position de commutation cible et, en l'absence d'un signal de détection pour l'atteinte de la position de commutation cible, est conçu pour émettre un signal de commutation pour le renvoi partiel ou complet de l'aiguillage (50) vers la position de commutation de départ, puis pour émettre un signal de commutation pour un nouveau déplacement de l'aiguillage (50) vers la position de commutation cible.

2. Sortie de comprimés selon la revendication 1, **caractérisée en ce que** la sortie de comprimés (46) présente au moins un canal de sortie (52, 54, 72).

3. Sortie de comprimés selon la revendication 2, **caractérisée en ce que** l'aiguillage (50) comporte au moins un drapeau d'aiguillage (58, 66) monté dans l'au moins un canal de sortie (52, 54, 72) de la sortie de comprimés (46) de manière à pouvoir pivoter entre la première position de commutation et l'au moins une deuxième position de commutation.

4. Sortie de comprimés selon l'une des revendications 2 ou 3, **caractérisée en ce qu'**au moins un canal de sortie (52, 54, 72) présente au moins une section (80, 82, 90, 92) s'élargissant dans la direction de sortie des comprimés (74, 76, 78), en s'élargissant de préférence de façon étagée.

5. Sortie de comprimés selon les revendications 3 et 4, **caractérisée en ce que** dans la direction de sortie des comprimés (74, 76, 78), l'au moins une section à élargissement (80, 82, 90, 92) se trouve en amont, de préférence immédiatement en amont de la région parcourue par le drapeau d'aiguillage (58, 66) lors du mouvement de pivotement.

6. Sortie de comprimés selon l'une des revendications 4 ou 5, **caractérisée en ce que** sur des parois opposées du canal de sortie (52, 54, 72), il est prévu respectivement au moins une section s'élargissant de façon étagée (80, 82, 90, 92).

7. Sortie de comprimés selon l'une des revendications 4 à 6, **caractérisée en ce que** l'élargissement de l'au moins une section à élargissement (80, 82, 90, 92) présente une largeur d'au moins 10 mm, avantageusement d'au moins 19 mm, plus avantageusement encore d'au moins 25 mm.

8. Presse à comprimés rotative, comportant un rotor apte à être mis en rotation par un entraînement rotatif, dans laquelle le rotor présente un guide de poinçons supérieur pour des poinçons supérieurs (14) de la presse à comprimés rotative et un guide de poinçons inférieur pour des poinçons inférieurs (16) de la presse à comprimés rotative, ainsi qu'un disque à matrice (10) disposé entre les guides de poinçons, dans laquelle les poinçons (14, 16) coopèrent avec des cavités du disque à matrice (10), comportant en outre un dispositif de remplissage (22), par le biais duquel un matériau de remplissage à comprimer est rempli dans les cavités (12) du disque à matrice (10), comportant en outre au moins un dispositif de pression supérieur (30) et au moins un dispositif de pression inférieur (30), lesquels coopèrent avec les poinçons supérieurs (14) et les poinçons inférieurs (16) pendant le fonctionnement pour presser le matériau de remplissage dans les cavités (12) du disque à matrice (10) sous forme de comprimés (74, 76, 78), comportant en outre un dispositif d'éjection (40), dans lequel des comprimés fabriqués dans les cavités (12) sont éjectés, **caractérisée en ce que** la presse à comprimés rotative comporte une sortie de comprimés (46) selon l'une des revendications précédentes, vers laquelle sont alimentés les comprimés (74, 76, 78) éjectés.

9. Procédé d'actionnement d'un aiguillage (50) d'une sortie de comprimés (46) d'une presse à comprimés, en particulier d'une presse à comprimés rotative, dans lequel l'aiguillage (50) est déplacé entre une première position de commutation, dans laquelle des comprimés (74, 76, 78) sont alimentés vers une première sortie de comprimés, et au moins une deuxième position de commutation, dans laquelle des comprimés (74, 76, 78) sont alimentés vers au moins une deuxième sortie de comprimés, dans lequel au moins un capteur détecte lorsque l'aiguillage (50) atteint la première position de commutation et/ou l'au moins une deuxième position de commutation, **caractérisé en ce que** lorsque l'atteinte de la position de commutation cible n'est pas détectée lors d'un déplacement de l'aiguillage (50) à partir d'une position de commutation de départ vers une position de commutation cible, l'aiguillage (50) est renvoyé partiellement ou complètement vers la position de commutation de départ et puis de nouveau déplacé vers la position de commutation cible.

10. Procédé selon la revendication 9, **caractérisé en ce que** le procédé est répété jusqu'à ce que l'atteinte de la position de commutation cible par l'aiguillage (50) soit détectée.

11. Procédé selon l'une des revendications 9 ou 10, **caractérisé en ce que** celui-ci est mis en œuvre avec une sortie de comprimés (46) selon l'une des revendications 1 à 7 ou avec une presse à comprimés rotative selon la revendication 8.
